# EUROPEAN PATENT SPECIFICATION

(11) **EP 1 768 584 B1**
(45) Date of publication and mention of the grant of the patent: **26.10.2011**
(21) Application number: 05768389.8
(22) Date of filing: 05.07.2005
(51) Int. Cl.: A61B 17/58

(54) **BONE PLATE SYSTEM WITH BONE SCREWS FIXED BY SECONDARY COMPRESSION**
KNOCHENPLATTENSYSTEM MIT KNOCHENSCHRAUBEN DIE DURCH SEKUNDÄRE KOMPRESSION FIXIERT WERDEN
SYSTÈME DE PLAQUE OSSEUSE MUNIE DE VIS FIXÉES PAR COMPRESSION SECONDAIRE

(30) Priority: 07.07.2004 US 586131 P; 21.01.2005 US 40779
(43) Date of publication of application: 04.04.2007
(73) Proprietor: DePuy Products, Inc., Warsaw, Indiana 46581-0988 (US)
(72) Inventor: ORBAY, Jorge, L., Coral Gables, FL 33156 (US); CASTANEDA, Javier, E., Miami, FL 33186 (US); GRAHAM, Robert, Miami, FL 33186 (US); CAVALLAZZI, Cesare, Miramar, FL 33027 (US)
(74) Representative: Gover, Richard Paul
(86) International application number: PCT/US2005/023855
(87) International publication number: WO 2006/014436

(56) References cited:
- WO-A-98/51226
- US-A- 5 616 144
- US-A- 6 152 927
- US-A1- 2003 078 583
- US-A1- 2004 097 950
- US-A1- 2005 004 574
- US-A1- 2005 004 574
- US-B1- 6 293 949

## Description

### BACKGROUND OF THE INVENTION

### 1. Field of the Invention

This invention relates broadly to orthopedic devices. More particularly, this invention relates to systems for locking bone screws relative to bone plates.

### 2. State of the Art

For various fractures of bones of the body, plating is a well known technique to impart the stabilization desirable for proper healing. In plating, a rigid, typically metal plate is placed on the outer surface of the bone across the fracture, and screws extend through the plate and are secured into the bone on either side of the fracture in a manner which permits the rigid plate to offer support to the bone during healing.

The screws include threads along a shaft adapted to engage cortical bone. Most commonly, the head portion of the screw is a standard screw head which provides a compressive force about a corresponding round screw hole of the plate as the fixator is threaded into the bone, thereby causing compression of the plate against the bone.

U.S. Pat. No. Re. 28,841 to Allgower describes a plate that is used with generally standard screws having heads with a convex undersurface. The plate includes oblong screw holes which each define at one end an upper ramped portion and a generally smaller radius of curvature about the ramped portion. In use, a hole is drilled into the bone through the screw hole adjacent the ramp and a screw is inserted into the drilled hole and rotated until the head of the screw contacts the ramp. Upon such engagement, there is displacement of the bone plate in a direction to move the ramped portion away from the screw and to cause the plate to apply pressure to maintain the bone parts together about the fracture in tight engagement.

More recently, particularly at the metaphysis of long bones though not limited thereto, there have been desirable results with threaded screws with threaded heads which threadably engage in threads in the plate to lock the screws relative to the plate and thereby limit compression of the plate relative to the bone. However, such systems do not provide the necessary control of compression between the plate and bone. Control over compressive forces is lost as soon as the threads of the head of the screw lock relative to the plate. Therefore, such a system provides sub-optimal stability.

Certain plates sold by Synthes of Paoli, PA are designed with a hole called a COMBI-HOLE™. The COMBI-HOLE™ is an elongated screw hole including two joined circular sections, each extending through approximately 250°. One of the circular sections is threaded and thus adapted to receive a screw with a threaded head at a fixed angle. When used as such, the system has the same lack of control over compression as discussed above. The other circular section is non-threaded and thus adapted to receive a standard non-threaded head and provide compression against the plate. In such section a screw can be angled slightly relative to the hole. However, the angle of the screw cannot be fixed.

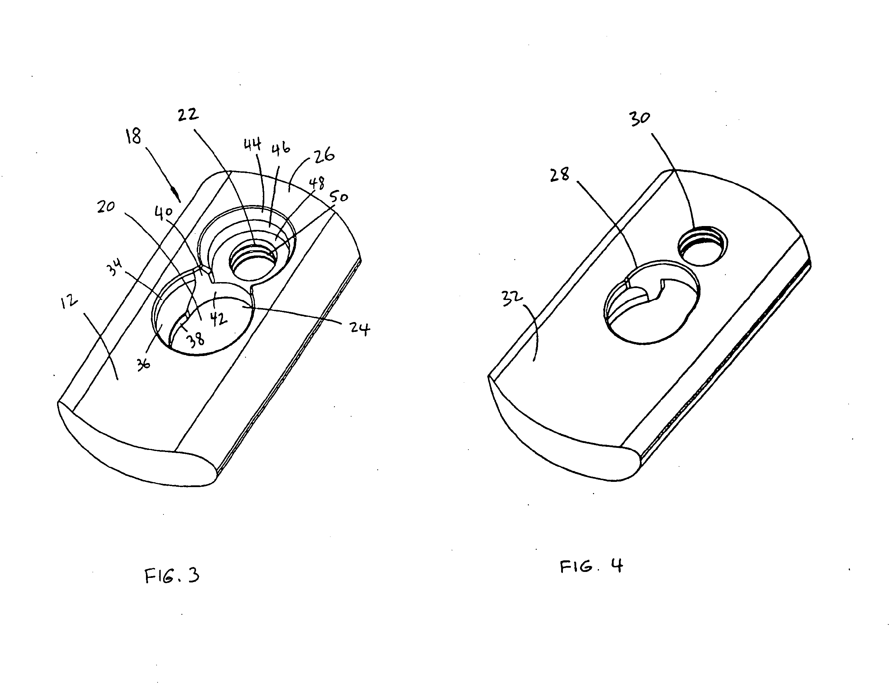

U.S. Pat. No. 5,549,612 to Yapp et al. teaches a system in which the angle of a screw can be fixed by use of a rotatable cam which contacts the head of the screw. However, because the cam is permanently mounted in an aperture in the plate and must be shaped to permit access for the bone screw, the cam cannot provide any downward force against the screw head, thereby limiting potential fixation. As such, if the cam rotates just a small amount from a locking angle, the fixation provided by the cam may be lost. Moreover, the shape of the cam (as shown in Figs. 4 and 4A of the patent) suggests that the cam applies an upward force against the screw head which disadvantageously counters the compressive force of the screw against the plate.

US-6293949-B1 discloses a device for stabilizing a portion of a spinal column including a longitudinal member sized to span at least two vertebral bodies formed from a shape memory material. A number of bone anchors are used to secure the longitudinal member to each of the vertebral bodies. A locking device engages adj acent bone anchors to prevent the bone anchors from loosening. For instance, the locking device may comprise a screw extending into the longitudinal member and into engagement with the heads of adjacent bone anchors.

WO-98/51226-A2 discloses a fixation system comprising a plate with a plurality of screw holes and a number of screws for fixing the plate to bones. A locking assembly is provided to lock one or more screws within respective screw holes. In one embodiment the locking assembly includes a washer overlapping one or more screw holes and a locking screw extending through the washer into the plate. Securing the locking screw to the plate causes the washer to be pressed against the heads of the bone screws.

US-2004/0097950-A1 discloses a system and method for securing a plate to a spinal column. The plate is arranged to extend across two or more adjacent vertebrae. The plate has a number of opening therethrough and a number of bone engaging fasteners, for instance bone screws, are insertable through the openings to secure the plate to the vertebrae. The plate defines a longitudinal axis extending along the length of the plate at its centre line. The bone screws are held in place by a retainer assembly extending along the longitudinal axis of the plate between adjacent pairs of bone screw holes. The retainer assembly comprises a washer extending along the length of the plate between adjacent pairs of bone screws. A plurality of apertures within the washer have countersunk edges such that as locking fasteners such as screws are inserted through the apertures into the plate, the washer slides along the longitudinal axis of the plate into sliding contact over the heads of the bone screws.

US-5616144 discloses an osteosynthesis plate system for securely fusing adjacent vertebrae. The plate includes a plurality of screw holes extending through the plate to seat bone screws fix on the plate to the vertebrae. A locking mechanism is disposed adjacent to each screw hole. The locking mechanism comprises a rotatable cam member permanently housed in an aperture within the plate.

It is therefore an object of the invention to provide a plate and screw system whereby the amount of compression between the plate and bone can be controlled completely by the surgeon.

It is another object of the invention to provide a plate and screw system in which each hole may be used in a fixed angle or variable angle manner with a screw.

It is a further object of the invention to provide a plate and screw system wherein when a screw is inserted in a variable angle mode, it can thereafter be locked in a desired angle.

It is an additional object of the invention to provide a plate and screw system which permits conventional screw-plate fixation.

In accordance with the present invention there is provided a bone plate system, comprising: a bone plate including a bone screw hole, an adjacent threaded set screw hole and a slot which extends from said bone screw hole and over said set screw hole; an element slidable within said slot along an axis extending through the axes of the bone screw hole and the set screw hole and including an elongate opening and a contact face; a bone screw having a head portion and a shaft arranged such that when the head portion is received in said bone screw hole said shaft extends below a lower surface of the bone plate; and a set screw having a circular head and a shaft, said shaft extending within said elongate opening and being threadably engaged in said set screw hole; wherein threadably inserting said set screw into said set screw hole causes the contact face of said element to be laterally driven against said head portion of said bone screw to compress said head portion of said bone screw between the contact face of said element and the wall of the bone screw hole opposite said element.

In accord with embodiments of the invention, the plate includes common openings adapted to receive the variable and fixed angle bone screws, both of which can be locked relative to the plate. More particularly, the plate may include a hole system for the bone screws and set screws. The hole system may define a threadless bone screw hole and an adjacent set screw hole. While the upper ends of the bone screw hole and set screw hole may define a common opening in the upper end of the plate, the lower ends of the respective holes may define distinct openings in plate. The bone screw hole may include an upper larger cylindrical portion, a central spherical portion, and a lower smaller cylindrical portion.

According to an embodiment of the invention, a locking washer is provided in a common opening of the plate. When the set screw is inserted into the plate, the set screw causes the washer to be longitudinally forced against the head of the bone screw to lock the bone screw in the plate. The side of the washer contacting the head of the bone screw may be spherically concave to provide maximum surface area contact to heads of such shape. In addition, the contact surfaces of the both the washer and the bone screw head may be provided with high friction textured surfaces to aid in locking the bone screw relative to the plate.

In accord with another mode of use of the plate and the variable angle screw, a hole is drilled for the screw along an axis which is offset towards the ramp and away from the circular center defined by the upper and lower cylindrical portions of the screw hole. The bone screw is driven into the hole until the head of the screw contacts the ramp portion which causes displacement of the plate by the distance required to seat the head in the concave spherical portions. This displacement applies pressure to maintain the bone parts together in tight engagement about a fracture. The set screw, and washer where provided, are then used to fix the level of compression and prevent loosening.

The plate and screws also allow conventional screw-plate fixation. That is, surgeons frequently desire to use different holes in different modes; i.e., either fixed angle or variable angle. Screws can be quickly inserted on each side of the fracture in a free-hand manner during fracture reduction. Thereafter, using other holes in the plate the surgeon can implant fixed angle screws and optionally other variable angle screws.

Additional objects and advantages of the invention will become apparent to those skilled in the art upon reference to the detailed description taken in conjunction with the provided figures.
Fig. 1 is a partial section side elevation view of a bone plating fixation system shown with a variable angle bone screw and corresponding set screw;
Fig. 2 is a partial section side elevation view of a bone plating fixation system shown with a fixed angle bone screw and corresponding set screw;
Fig. 3 is a top perspective view of a broken section of a plate showing a one hole system;
Fig. 4 is a bottom perspective view of a broken section of a plate showing the one hole system;
Fig. 5 is a partial section side elevation view of a bone plating fixation system shown with a variable angle bone screw used in a plate- displacement mode of operation;
Fig. 6 is a perspective longitudinal section view of an embodiment of a bone plating fixation system in accordance with the present invention shown in a non-locked configuration;
Fig. 7 is a perspective view of the bone plating fixation system of Figure 6, shown in the non-locked configuration;
Fig. 8 is a perspective longitudinal section view of the bone plating fixation system of Figure 6, shown in a locked configuration;
Fig. 9 is a perspective view of the bone plating fixation system of Figure 6, shown in the locked configuration;
Fig. 10 is a perspective longitudinal section view of a second embodiment of a bone plating fixation system in accordance with the present invention, shown in an unlocked configuration;
Fig. 11 is a perspective longitudinal section view of the bone plating fixation system of Figure 10, shown in a locked configuration;
Fig. 12 is a perspective view of the bone plating fixation system of Figure 10, shown in the locked configuration;
Fig. 13 is a perspective longitudinal section view of a third embodiment of a bone plating fixation system in accordance with the present invention shown in a locked configuration;
Fig. 14 is a top perspective view of the bone plating fixation system of Figure 13, shown in the locked configuration; and
Fig. 15 is a perspective longitudinal section view of a bone plating fixation system, shown in a locked configuration.

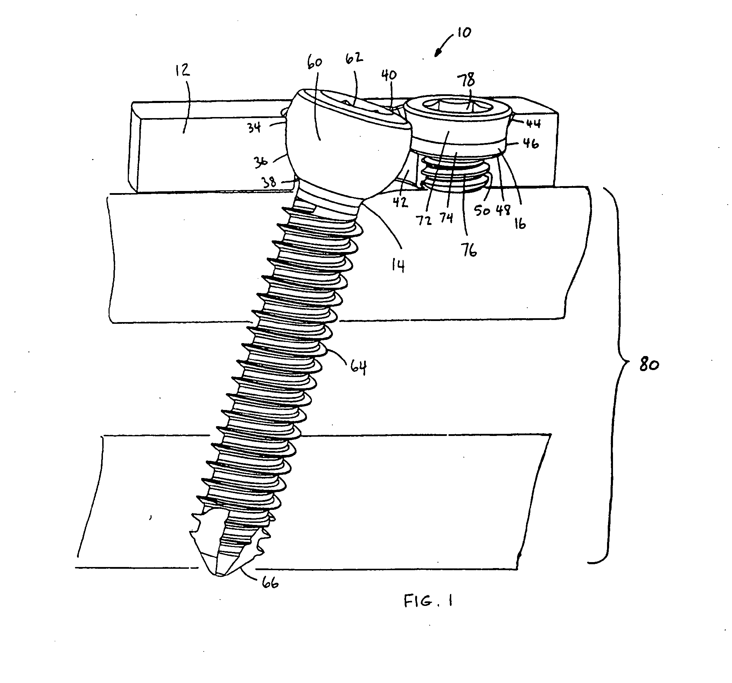

Figures 1 to 5 and 15 illustrate bone plating systems useful for understanding the present invention but not falling within the scope of the claims.

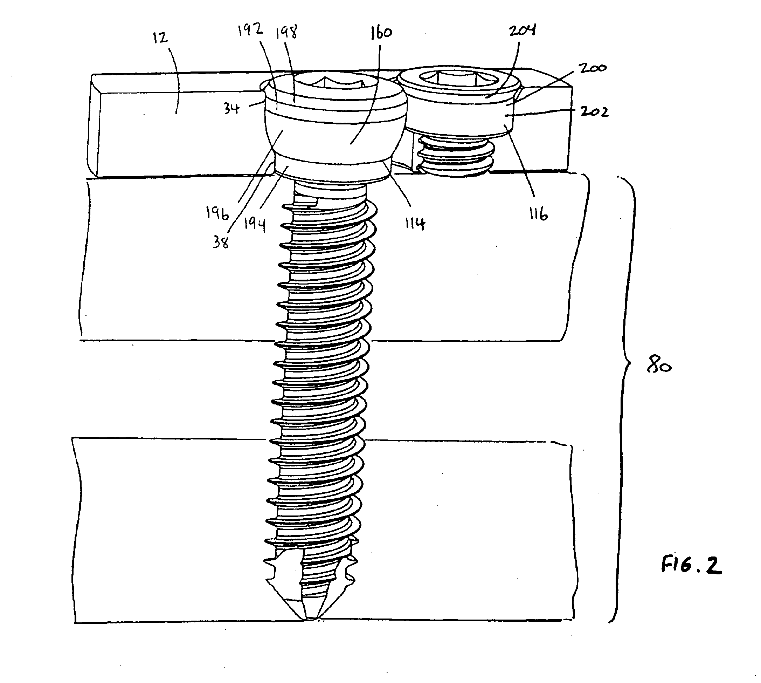

Turning now to Figs. 1 and 2, a bone plating system 10 includes a plate 12, and one or both of bone screws 14, 114, and set screws 16, 116. The plate 12 can be any orthopedic plate which has application in providing compression or other stabilization to bone, including but not limited to, plates for fractures of the diaphysis and/or metaphysis of long bones, plates for placement on the mandible or other portions of the skull, and plates for osteosynthesis, particularly along the vertebrae.

Referring to Figs. 3 and 4, the plate 12 includes a plurality of a hole systems 18 for the bone screws and set screws. Depending upon the application, such hole systems can reside on each side of a fracture. More particularly, each hole system 18 defines a threadless bone screw hole 20 and an adjacent threaded set screw hole 22. While the upper ends of the bone screw hole 20 and set screw hole 22 define a common opening 24 in the upper surface 26 of the plate 12, the lower ends of the respective holes preferably define distinct openings 28, 30 in the lower surface 32 of the plate.

Referring to Figs. 1 and 3, the bone screw hole 20 includes an upper larger cylindrical portion 34, a central spherical portion 36, and a lower smaller cylindrical portion 38. The cylindrical portions 34 and 38 alternatively may be frustoconical in shape. Adjacent the set screw hole 22, the bone screw hole 20 includes an upper ramp portion 40 for imparting compression across a fracture, as discussed below, and a lower portion 42 with a smaller radius of curvature than cylindrical portion 38. The set screw hole 22 includes an upper conical portion 44, a central cylindrical portion 46, a shelf 48, and a lower portion 50 provided with machine threads.

The bone screws preferably include both variable angle screws 14 and fixed angle screws 114. Referring to Fig. 1, each variable angle screw 14 has a head portion 60 which is spherically curved and includes an upper recess 62, e.g., hex slot, for a driver, and a threaded shaft 64 preferably including a self-drilling fluted tip 66. The set screw 16 for use with the variable angle screw 14 includes a head 70 having an upper spherically concave portion 72, matched to the radius of curvature of head portion 60, and a lower cylindrical portion 74. The set screw 16 also includes a shaft 76 with machine threads sized to engage in threads 50 of the set screw holes. The set screw further includes an upper recess 78, e.g., a hex slot for rotationally driving the set screw. In accord with an aspect of the invention, it is preferable that upper recesses 62 and 78 have the same shape and dimension such that each can be driven by the same tool.

In use, a hole is drilled in a desired angle through the bone screw hole of the plate 12 and into bone for the variable angle screw 14. The bone screw 14 is then driven into the bone 80, e.g., the diaphyseal bone shown, through the hole. Alternatively, the bone screw is driven at the desired angled without pre-drilling. Once the head 60 of the screw 14 reaches the plate 12, the screw 14 is driven until the desired amount of the compression is created between the plate 12 and bone 80. This generally requires at least 180° of rotation, and often several complete rotations of the screw. The bone screw 14 then can be fixed relative to the plate 12 by insertion of the set screw 16 into the set screw hole 22. The set screw 16 is driven until sufficient compressive forces are developed between the spherically concave portion 72 of the set screw 16 and the spherically concave portion 36 of the opposite wall of the bone screw hole 20 so as to lock the head 60 of the bone screw 14 therebetween relative to the plate 12. Thus, the variable angle bone screw 16 can be used to compress the plate 12 against the bone 80 at a desired angle and can then be locked at such angle and positioning without later loosening, as the bone screw 14 is held in relation to the plate 12 by the set screw 16.

Referring back to Fig. 2, each fixed angle bone screw 114 is substantially similar to the variable angle bone screw 14, with distinction in the shape of the head 160. The head 160 includes upper and lower cylindrical portions 192, 194, and a central spherical portion 196 that matches the dimensions and radius of the bone screw hole. In addition, the upper edge of the head 160 is provided with a conical taper 198. The set screw 116 for use with the fixed angle bone screw 114 is substantially similar to the set screw 16, with distinction to the shape of its head 200. The head 200 of the set screw 116 includes a lower cylindrical portion 202 and an upper conical lip 204.

In use, the fixed angle bone screw 114 is driven (with or without pre-drilling a pilot hole) at a location substantially concentric with the circular center defined by the upper and lower cylindrical portions 34, 38, of the bone screw hole 20 (Fig. 3). The head 160 of the screw 114 seats within the space defined by portions 34, 36, 38 and as head 160 is driven toward the bone, the plate is compressed against the bone. The bone screw 114 can be driven until a desired compression is effected. The bone screw is maintained in a fixed angle (i.e., normal to the plate) by being subject to three points of fixation. Upon insertion of the set screw 116 into screw hole 22, upper and lower cylindrical portions 34, 38 of the screw hole 20 and cylindrical portion 202 of the set screw 116 provide the three points of fixation. In addition, to fix the level of compression and to prevent any loosening that may occur through micromotion, the set screw 116 is driven until the conical lip 204 creates sufficient force against the head 160 of the bone screw 114 at taper 198 so as to prevent any backing off of the bone screw 114. That is, the set screw 116 locks the bone screw 114 in place.

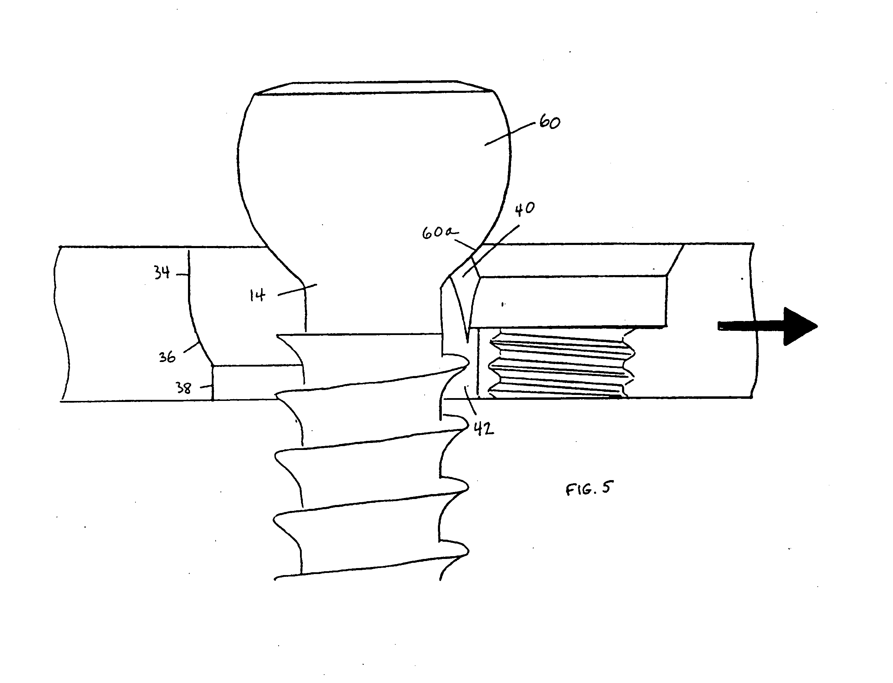

Turning now to Fig. 5, in accord with another mode of using the plate and the variable angle screw 14, a hole is drilled for the screw 14 along an axis normal to the plate which is offset towards the ramp 40 and generally concentric with cylindrical portion 42; i.e., away from the circular center defined by the upper and lower cylindrical portions 34, 38 of the screw hole 20. The bone screw 14 is then driven into the hole until the lower portion 60a of the spherical head 60 of the screw contacts the ramp 40 and thus causes displacement of the plate 12 in the direction of the arrow by the distance required to seat the head 60 in the concave spherical portion 36. This displacement applies pressure which maintains bone parts together about a fracture in tight engagement. The set screw 16 is then used, as previously described, to fix the level of compression and lock the screw to the plate 12 to prevent loosening.

The plate and screws can also be used in a manner which is known to those familiar to screw-plate fixation. That is, surgeons frequently desire to use different holes in different modes; i.e., either fixed angle or variable angle. By way of example, screws can be quickly inserted on each side of the fracture in a free-hand variable angle manner during fracture reduction. Thereafter, using other holes in the plate and careful alignment, e.g., drilling pilot holes with a drill guide, the surgeon can implant fixed angle screws.

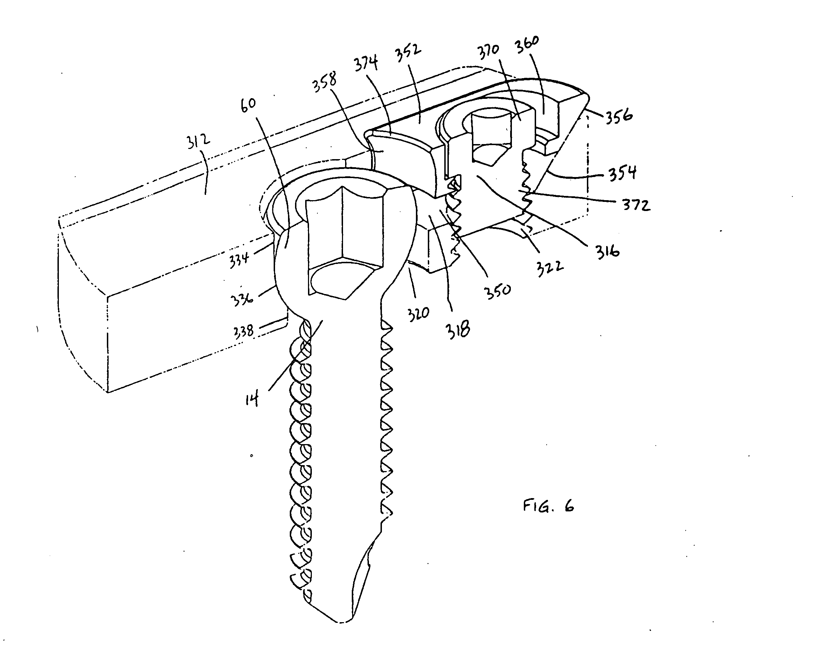

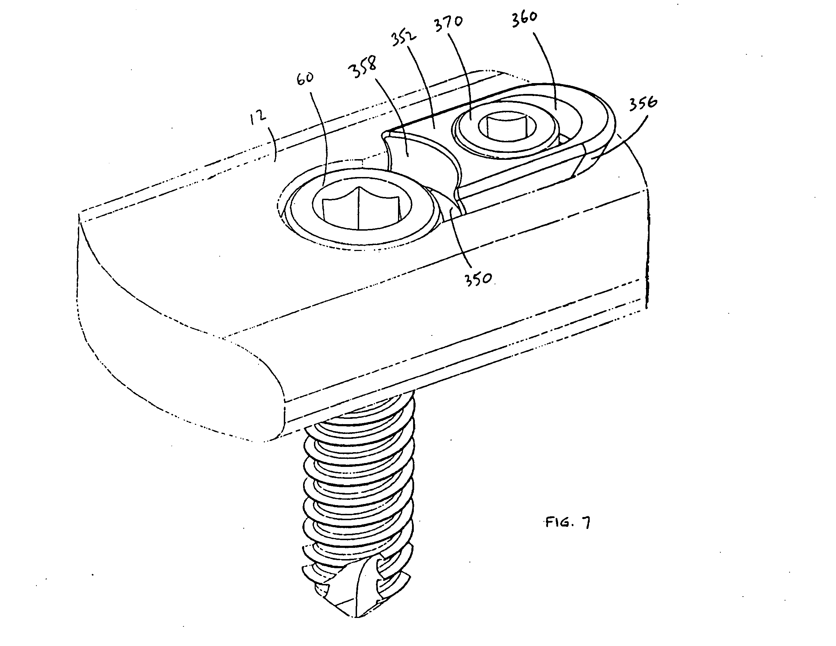

Turning now to Figs. 6 and 7, a first embodiment of the invention is shown. The system includes a plate 312 having a hole system 318 which include a threadless bone screw hole 320, a slot 350 for a washer 352, and a set screw hole 322 for set screw 316. The bone screw hole 320, as described above, includes an upper larger cylindrical portion 334, a central spherical portion 336, and a lower smaller cylindrical portion 338. A bone screw, e.g., screw 14 described above, can be provided in the screw hole 320. The slot 350 includes a rear camming ramp 354. The washer 352 includes a cam surface 356 which rides on the camming ramp 354, a substantially spherically concave contact face 358 directed toward the bone screw hole 320, and an oblong slot 360 for the head 370 and shaft 372 of the set screw 316. The set screw hole 322 in the plate 312 is preferably uniform in diameter. The set screw head 370 is preferably cylindrical, and the shaft 372 of the set screw 316 threadably engages the set screw hole 322. The washer 352 and set screw 316 are preferably pre-installed in the slot 350 and set screw hole 322 (as shown in Fig. 6) prior to positioning the plate 312 on the bone (e.g., at the factory or by an operating room technician).

In use, a hole is drilled in a desired angle through the bone screw hole 320 and into bone for the variable angle screw 14. The bone screw 14 is then driven into the bone through the hole. Alternatively, the bone screw is driven at the desired angled without pre-drilling. As yet another alternative, a fixed angle screw 114 (Fig. 2) may be inserted into a hole drilled concentric with the bone screw hole 320.

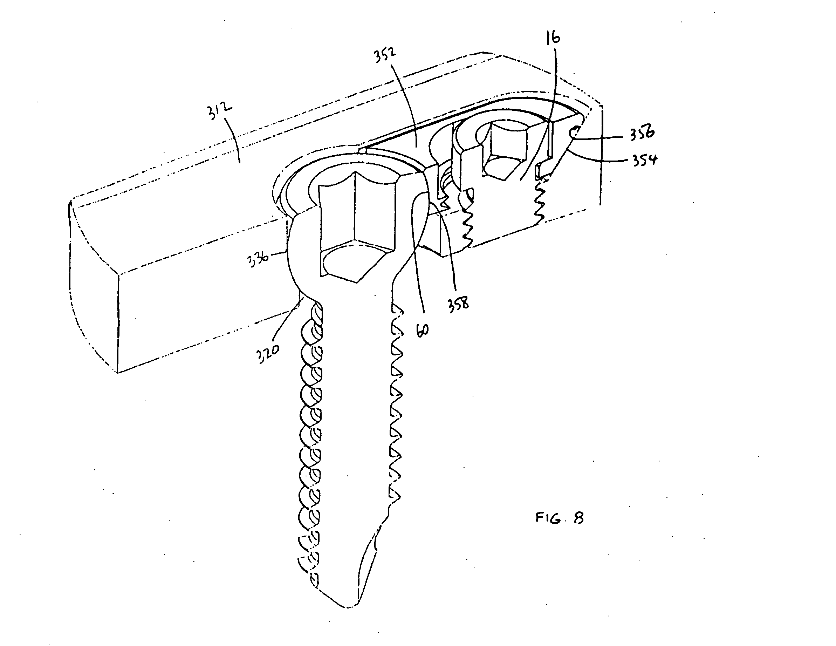

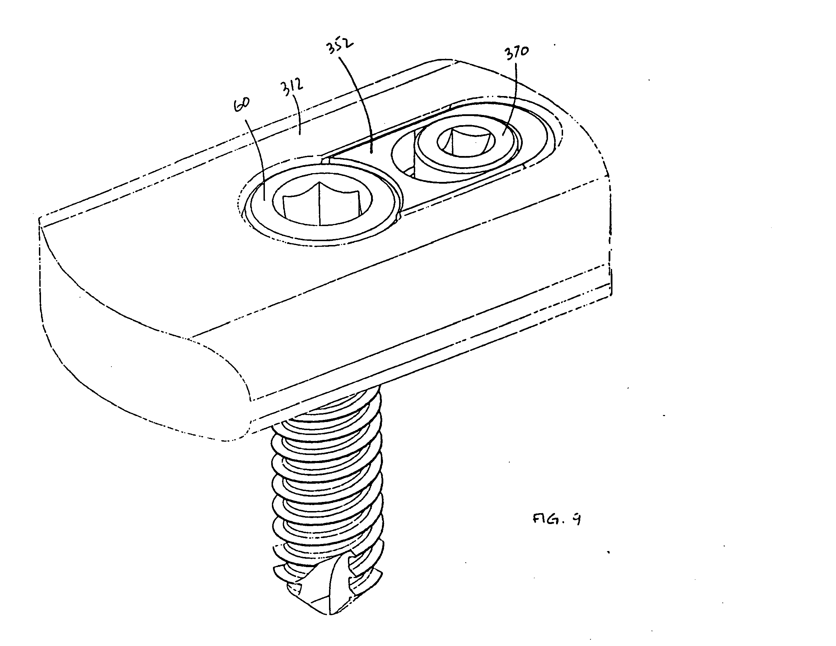

Once the head 60 of the screw 14 reaches the plate 312, the screw 14 is driven until the desired amount of the compression is created between the plate and bone. The relation of the bone screw 14 relative to the plate, including its angular position, can then be fixed relative to the plate 312 by rotating the set screw 316 into the set screw hole 322 to cause the washer 352 to be forced down and forward, along rear camming ramp 354. Referring to Figs. 8 and 9, this results in contact face 358 being driven toward and into the head 60 of the screw. The set screw 16 is rotated until sufficient compressive forces are developed between the contact face 358 of the washer and the spherically concave portion 336 of the opposite wall of the bone screw hole 320 so as to lock the head 60 of the bone screw 14 therebetween. The amount of rotation is generally at least 180° which provides suitable travel for the head 370 of the set screw and sufficient engagement between the shaft 372 of the set screw and the plate. In addition, the bone screw hole may 320 be slightly eccentric or the contact face 358 may be slightly aspheric so as to cause three points of circumferential contact when the washer is driven toward the bone screw for even greater stability. It is appreciated that by driving a washer 352 toward the bone screw head 60 significantly greater surface area is available to develop larger compressive forces relative to the use of a set screw. Further, by longitudinally driving the washer, the contact face 358 and outer surface of the bone screw head 60 can be provided with a textured high friction surface, discussed below, which is not generally practical when two surfaces must be rotated relative to each other. The above system can similarly be used with a fixed angle bone screw 114 (Fig. 2).

It is also noted that upper edge 374 of the of contact face 358 may be further beveled and/or contoured to function as a ramp to enable displacement of the plate 312; i.e., to function similarly to ramp 40, described above.

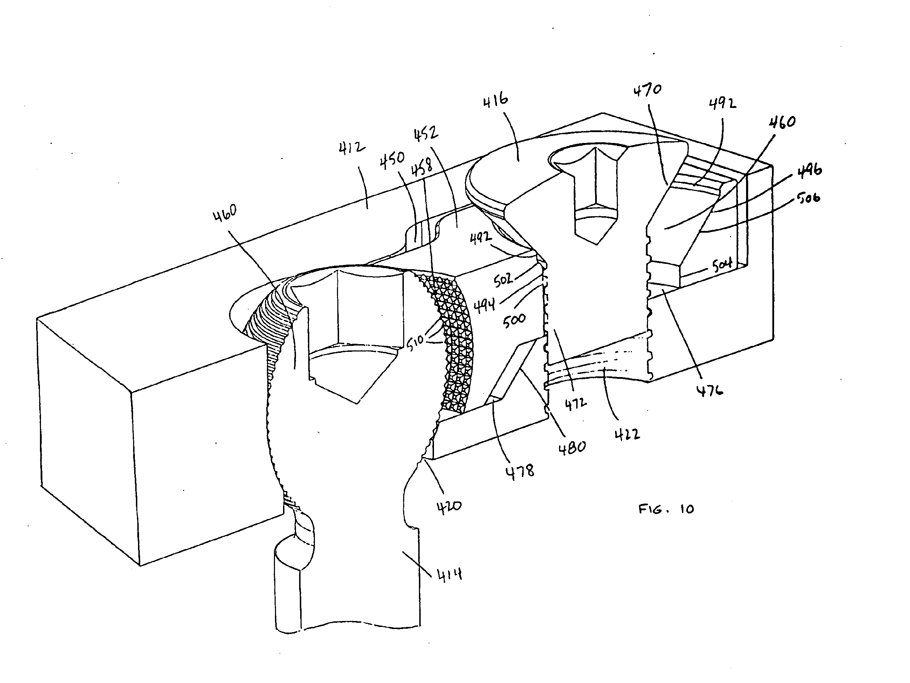

Referring now to Fig. 10, a second embodiment of the invention, similar to the first embodiment, is shown. The plate 412 includes a hole system defining a bone screw hole 420, a washer slot 450, and a set screw hole 422. The washer slot 450 includes upper and lower portions 476, 478 with a step or ramp 480 therebetween. The washer 452 includes a spherically concave textured contact face 458 (e.g., with knurls), an opening 460 defined by a circular upper rim 492, and front and rear walls 494, 496 of equal height. The front wall 494 has lower portion 500 with a constant radius which is approximately 80% of the height of the front wall and an upper conically beveled portion 502 which extends approximately 10% of the height of the front wall. The rear wall 496 has a lower portion 504 with the constant radius which is approximately 25% of the height of the rear wall and an upper conically beveled portion 506 which extends approximately 65% of the height of the rear wall. Lower portions 500 and 504 have the same radius, though their radial centers are longitudinally offset along the longitudinal axis of the hole system. In addition, beveled portions 502 and 506 are beveled at the same angle. The set screw 416 includes a head 470 with a conically sloped undersurface which functions as a cam, as described below. The bone screw 414 is similar to screw 14, but the head 460 of the screw includes a textured surface, e.g., with circumferential grooves 510.

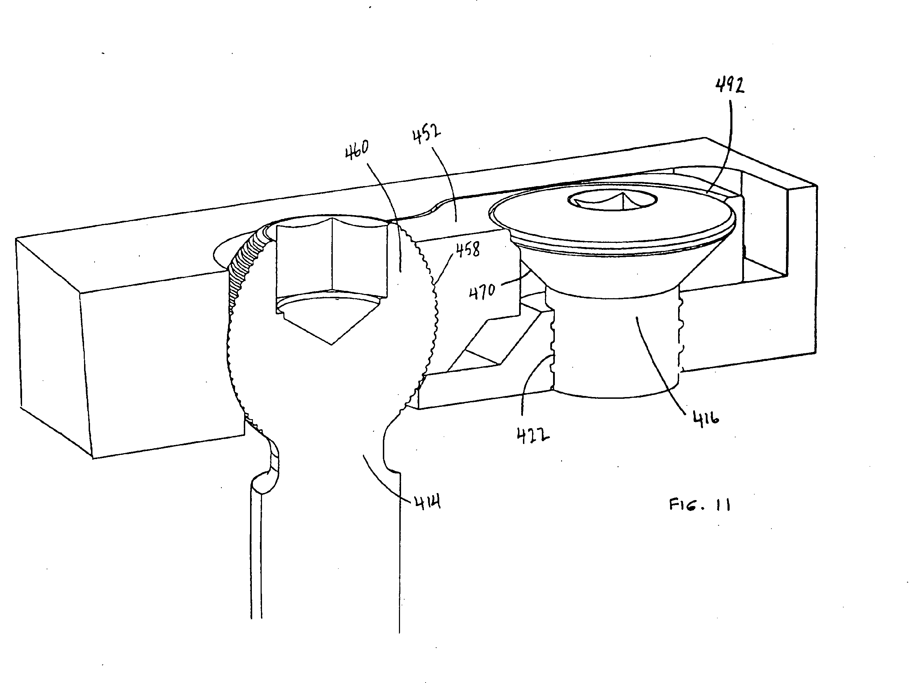

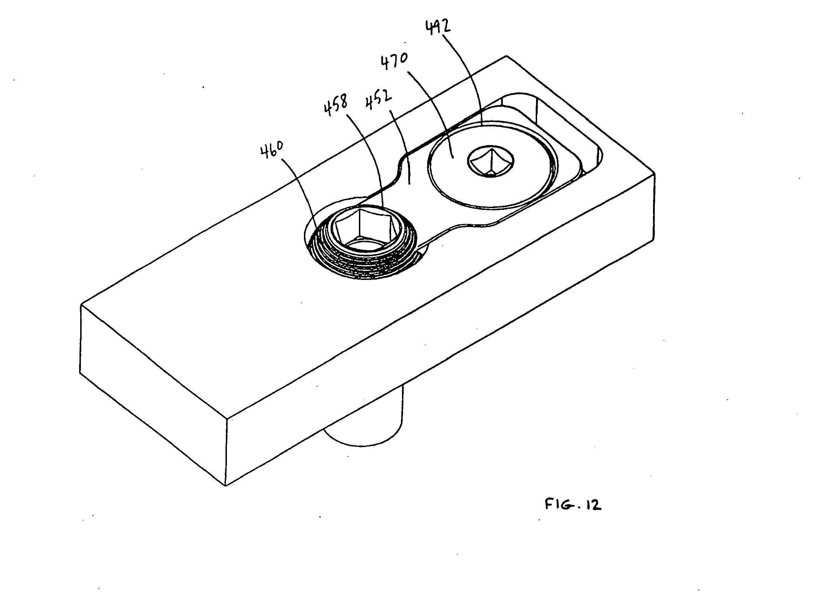

In use, the system is operated in a substantially similar manner to that described above with respect to Figs. 6-9. The distinctions are as follows. In the initial positions of the set screw 416 and washer 452, shown in Fig. 10, the shaft 472 of the set screw 416 abuts lower portion 500 of the front wall 494 and the conical undersurface of the head 470 of the set screw contacts the edge of the rim 492. Referring to Figs. 11 and 12, as the set screw 416 is driven into the hole 422, the slope on the undersurface of the head 470 forces the washer 452 toward the head 460 of the bone screw 414. Once the washer has moved toward the head of the bone screw, the top of head 470 of the set screw fully seats within the circumferential rim 492, locking the washer 452 in place. In addition, the textured surfaces on the contact face 458 and head 460 operate to create a secure interlock.

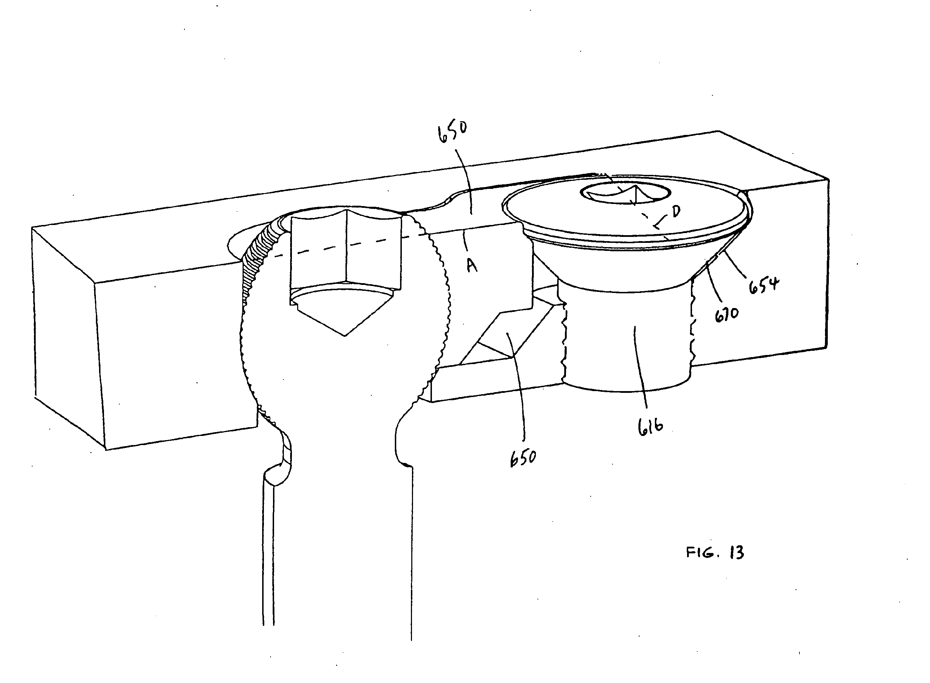

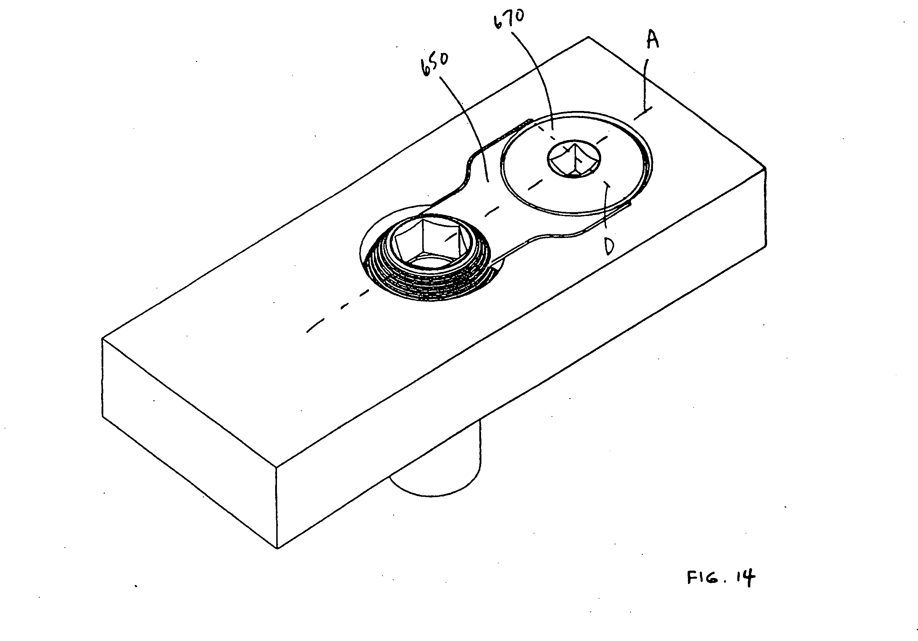

Referring now to Figs. 13 and 14, a third embodiment of the invention, substantially similar to the second embodiment, is shown. The distinctions are as follows. The slot 650 is shorter and includes a rear wall 654 which is sloped to correspond to the conical shape of the undersurface of the head 670 of the set screw 616. The washer 650 does not include a rear wall, and preferably terminates at or forward to the diameter D of the set screw head which is transverse to the longitudinal axis A of the hole system. This construction permits a relatively smaller washer and smaller overall locking system, but functions substantially the same as the previous embodiment.

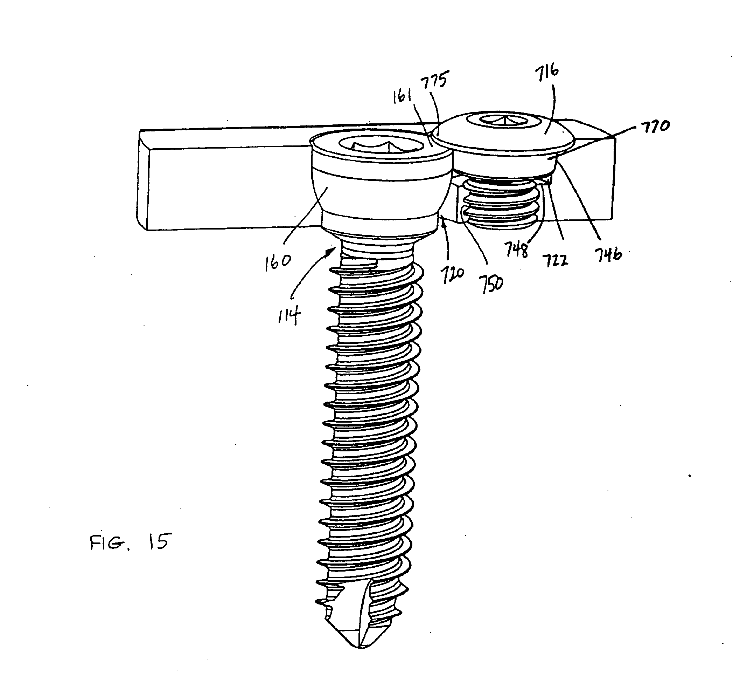

Turning now to Fig. 15, a bone plate system similar to the bone plate system shown in Fig. 2 is provided. The set screw hole 722 includes an upper frustoconical (or cylindrical) portion 746, a shelf 748, and a lower portion 750 provided with machine threads (but no upper conically flared portion in distinction from the embodiment of Fig. 2). The set screw 716 includes a head 770 with an upper lateral lip 775.

After insertion of a fixed angle bone screw 114 into the bone screw hole 720 as described above, the set screw 716 is inserted in the set screw hole 722. The bone screw 114 can be driven until a desired compression is effected. The bone screw 114 is maintained in a fixed angle by the corresponding shapes of the 160 head of the bone screw and the above described contour of the bone screw hole 720 (see description with respect to bone screw hole 20). In order to lock the bone screw 114 within the bone screw hole 720, the set screw 116 is driven until the lateral lip 775 provides compression against the top surface 161 of the head 160 of the bone screw 114.

While various bone screw holes have been described that can accommodate both fixed angle and variable angle screws, it is appreciated that bone screw holes may be provided than can accommodate only a fixed angle or variable angle screw which is then locked to the plate. For example, for a fixed angle screw, the screw hole may be a single frustoconical hole which prevents travel of a frustoconical bone screw head through the hole. Alternatively, the hole may be a stepped cylinder which provides similar advantage to a correspondingly shaped head of a bone screw.

There have been described and illustrated herein embodiments of a bone plating system and method of using the same. While particular embodiments of the invention have been described, it is not intended that the invention be limited thereto, as it is intended that the invention be as broad in scope as the art will allow and that the specification be read likewise. It will therefore be appreciated by those skilled in the art that yet other modifications could be made to the provided invention without deviating from its scope as claimed.

## Claims

1. A bone plate system, comprising:
a bone plate (312, 412) including a bone screw hole (320, 420), an adjacent threaded set screw hole (322, 422) and a slot (350, 450) which extends from said bone screw hole (320, 420) and over said set screw hole (322, 422);
an element (352, 452, 650) slidable within said slot (350, 450) and including an elongate opening (360, 460) and a contact face (358, 458);
a bone screw (14, 414) having a head portion (60, 460) and a shaft arranged such that when the head portion (60, 460) is received in said bone screw hole (320, 420) said shaft extends below a lower surface of the bone plate (312, 412); and
a set screw (316, 416, 616) having a circular head (370, 470, 670) and a shaft (372,472), said shaft (372, 472) extending within said elongate opening (360, 460) and being threadably engaged in said set screw hole (322, 422);
wherein threadably inserting said set screw (316, 416, 616) into said set screw hole (322, 422) causes the contact face (358, 458) of said element (352, 452, 650) to be laterally driven against said head portion (60, 460) of said bone screw (14, 414) to compress said head portion (60, 460) of said bone screw (14, 414) between the contact face (358,458) of said element (352, 452, 650) and the wall of the bone screw hole (320, 420) opposite said element (352, 452, 650), **characterized in that**;
said element (352, 452, 650) is slidable within said slot (350, 450) along an axis extending through the axes of the bone screw hole (320, 420) and the set screw hole (322, 422)

2. A bone plate system according to claim 1, wherein:
said slot (350) includes a rear cam surface (356) opposite said bone screw hole (320) and when said set screw (316) is driven into said plate (312) said rear cam surface (356) forces said element (352) toward said bone screw hole (320).

3. A bone plate system according to claim 1, wherein:
said set screw (416, 616) includes a cam surface which drives said element (452, 650) against said bone screw (414).

4. A bone plate system according to claim 1, wherein:
said element (352, 452) surrounds said shaft (372, 472) of said set screw (316, 416).

5. A bone plate system according to claim 1, wherein:
said element (650) is provided only partially around said shaft of said set screw (616).

6. A bone plate system according to claim 1, wherein:
said contact face (358, 458) is provided with a textured surface.

7. A bone plate system according to claim 6, wherein said head portion (60, 460) of the bone screw (14, 414) has a textured surface which when contacted by said contact face (358, 458) under force creates an interlock between the bone screw (14, 414) and element (352, 452, 650)

8. A bone plate system according to claim 7, wherein:
said textured surfaces of said head portion (60, 460) of said bone screw (14, 414) and said contact face (358, 458) are different.

## Patentansprüche

1. Knochenplattensystem, das aufweist:
eine Knochenplatte (312, 412), die ein Knochenschraubenloch (320, 420), ein benachbart liegendes, mit Gewinde versehenes Stellschraubenloch (322, 422) und einen Schlitz (350, 450), der sich von dem Knochenschraubenloch (320, 420) und über das Stellschraubenloch (322, 422) erstreckt, umfasst;
ein Element (352, 452, 650), das innerhalb des Schlitzes (350, 450) verschieblich ist und eine längliche Öffnung (360, 460) und eine Kontaktfläche (358, 458) umfasst;
eine Knochenschraube (14, 414) mit einem Kopfbereich (60, 460) und einem Schaft, der so angeordnet ist, dass, wenn der Kopfbereich (60, 460) in dem Knochenschraubenloch (320, 420) aufgenommen ist, sich der Schaft unterhalb einer unteren Fläche der Knochenplatte (312, 412) erstreckt; und
eine Stellschraube (316, 416, 616) mit einem kreisförmigen Kopf (370, 470, 670) und einem Schaft (372, 472), wobei sich der Schaft (372, 472) innerhalb der länglichen Öffnung (360, 460) erstreckt und im Gewindeeingriff in dem Stellschraubenloch (322, 422) liegt;
wobei das Einschrauben der Stellschraube (316, 416, 616) in das Stellschraubenloch (322, 422) bewirkt, dass die Kontaktfläche (358, 458) des Elements (352, 452, 650) seitlich gegen den Kopfbereich (60,460) der Knochenschraube (14, 414) getrieben wird, um den Kopfbereich (60, 460) der Knochenschraube (14, 414) zwischen der Kontaktfläche (358, 458) des Elements (352, 452, 650) und der Wand des Knochenschraubenlochs (320, 420) gegenüber dem Element (352, 452, 650) zusammenzudrücken,
**dadurch gekennzeichnet, dass**
das Element (352, 452, 650) innerhalb des Schlitzes (350, 450) entlang einer Achse, die sich durch die Achsen des Knochenschraubenloches (320, 420) und des Stellschraubenloches (322, 422) erstreckt, verschieblich ist.

2. Knochenplattensystem nach Anspruch 1, bei dem:
der Schlitz (350) eine rückwärtige Kurvenfläche (356) gegenüber dem Knochenschraubenloch (320) umfasst, und wenn die Stellschraube (316) in die Platte (312) getrieben wird, die rückwärtige Kurvenfläche (356) das Element (352) auf das Knochenschraubenloch (320) zu treibt.

3. Knochenplattensystem nach Anspruch 1, bei dem:
die Stellschraube (416, 616) eine Kurvenfläche umfasst, die das Element (450, 650) gegen die Knochenschraube (414) treibt.

4. Knochenplattensystem nach Anspruch 1, bei dem:
das Element (352, 452) den Schaft (372, 472) der Stellschraube (316, 416) umgibt.

5. Knochenplattensystem nach Anspruch 1, bei dem:
das Element (650) lediglich teilweise um den Schaft der Stellschraube (616) herum vorgesehen ist.

6. Knochenplattensystem nach Anspruch 1, bei dem:
die Kontaktfläche (358, 458) mit einer texturierten Oberfläche versehen ist.

7. Knochenplattensystem nach Anspruch 6, bei dem:
der Kopfbereich (60, 460) der Knochenschraube (14, 414) eine texturierte Oberfläche hat, die,
wenn sie in Kontakt mit der Kontaktfläche (358, 458) kommt, unter Kraft eine Verriegelung zwischen der Knochenschraube (14, 414) und dem Element (352, 452, 650) erzeugt.

8. Knochenplattensystem nach Anspruch 7, bei dem:
die texturierten Oberflächen des Kopfbereiches (60, 460) der Knochenschraube (14, 414) und der Kontaktfläche (358, 458) unterschiedlich sind.

## Revendications

1. Système de plaque osseuse, comprenant :
✔ une plaque osseuse (312, 412) incluant un trou de vis à os (320, 420), un trou de vis de fixation fileté adjacent (322, 422) et une fente (350, 450) qui s'étend à partir dudit trou de vis à os (320, 420) et au-dessus dudit trou de vis de fixation (322, 422) ;
✔ un élément (352, 452, 650) pouvant coulisser à l'intérieur de ladite fente (350, 450) et incluant une ouverture allongée (360, 460) et une face de contact (358, 458) ;
✔ une vis à os (14, 414) ayant une partie de tête (60, 460) et une tige agencée de telle sorte que lorsque la partie de tête (60, 460) est reçue dans ledit trou de vis à os (320, 420), ladite tige s'étend en dessous d'une surface inférieure de la plaque osseuse (312, 412) ; et
✔ une vis de fixation (316, 416, 616) ayant une tête circulaire (370, 470, 670) et une tige (372, 472), ladite tige (372, 472) s'étendant à l'intérieur de ladite ouverture allongée (360, 460) et étant en prise par filetage dans ledit trou de vis de fixation (322, 422) ;
dans lequel l'insertion par filetage de ladite vis de fixation (316, 416, 616) dans ledit trou de vis de fixation (322, 422) provoque l'entraînement latéral de la face de contact (358, 458) dudit élément (352, 452, 650) contre ladite partie de tête (60, 460) de ladite vis à os (14, 414) pour comprimer ladite partie de tête (60, 460) de ladite vis à os (14, 414) entre la face de contact (358, 458) dudit élément (352, 452, 650) et la paroi du trou de vis à os (320, 420) à l'opposé dudit élément (352, 452, 650), **caractérisé en ce que**
ledit élément (352, 452, 650) peut coulisser à l'intérieur de ladite fente (350, 450) le long d'un axe s'étendant à travers les axes du trou de vis à os (320, 420) et du trou de vis de fixation (322, 422).

2. Système de plaque osseuse selon la revendication 1, dans lequel ladite fente (350) comprend une surface de came arrière (356) à l'opposé dudit trou de vis à os (320) et lorsque ladite vis de fixation (316) est introduite dans ladite plaque (312), ladite surface de came arrière (356) force ledit élément (352) vers ledit trou de vis à os (320).

3. Système de plaque osseuse selon la revendication 1, dans lequel ladite vis de fixation (416, 616) comprend une surface de came qui entraîne ledit élément (452, 650) contre ladite vis à os (414).

4. Système de plaque osseuse selon la revendication 1, dans lequel ledit élément (352, 452) entoure ladite tige (372, 472) de ladite vis de fixation (316, 416).

5. Système de plaque osseuse selon la revendication 1, dans lequel ledit élément (650) n'est prévu que partiellement autour de ladite tige de ladite vis de fixation (616).

6. Système de plaque osseuse selon la revendication 1, dans lequel ladite face de contact (358, 458) est dotée d'une surface texturée.

7. Système de plaque osseuse selon la revendication 6, dans lequel ladite partie de tête (60, 460) de la vis à os (14, 414) a une surface texturée qui, lorsqu'elle entre en contact avec ladite face de contact (358, 458) sous la force,
crée un interverrouillage entre la vis à os (14, 414) et l'élément (352, 452, 650).

8. Système de plaque osseuse selon la revendication 7, dans lequel lesdites surfaces texturées de ladite partie de tête (60, 460) de ladite vis à os (14, 414) et ladite face de contact (358, 458) sont différentes.
